# EUROPÄISCHE PATENTSCHRIFT

(11) **EP 3 801 448 B1**
(45) Veröffentlichungstag und Bekanntmachung des Hinweises auf die Patenterteilung: **10.07.2024**
(21) Anmeldenummer: 19725760.3
(22) Anmeldetag: 27.05.2019
(51) Int. Cl.: A61K 8/37, A61K 8/44, A61K 8/46, A61Q 5/02, A61Q 5/12, A61Q 19/10

(54) **WACHSDISPERSIONEN MIT KONDITIONIERENDEN EIGENSCHAFTEN**
WAX DISPERSIONS WITH CONDITIONING PROPERTIES
DISPERSIONS DE CIRE À PROPRIÉTÉS DE CONDITIONNEMENT

(30) Priorität: 30.05.2018 EP 18174997
(43) Veröffentlichungstag der Anmeldung: 14.04.2021
(73) Patentinhaber: BASF SE, 67056 Ludwigshafen am Rhein (DE)
(72) Erfinder: NIEENDICK, Claus, 40589 Düsseldorf-Holthausen (DE); MAUER, Werner, 40589 Düsseldorf-Holthausen (DE); BECKER, Anke, 40589 Düsseldorf-Holthausen (DE); CORNELSEN, Sybille, 40789 Monheim (DE)
(74) Vertreter: BASF IP Association
(86) Internationale Anmeldenummer: PCT/EP2019/063639
(87) Internationale Veröffentlichungsnummer: WO 2019/228975

(56) Entgegenhaltungen:
- WO-A1-02/05781
- DE-A1- 10 150 725
- DE-A1- 4 232 506
- DE-C2- 19 511 572

## Beschreibung

### Gebiet der Erfindung

Die Erfindung betrifft trübe Wachsdispersionen enthaltend ausgewählte Ethylenglykolfettsäureester als Wachskörper, Fettsäurepartialglyceride, anionische und amphotere Tenside, die konditionierende Eigenschaften aufweisen sowie die Verwendung derartiger Wachsdispersionen als Trübungs- und/oder Konditioniermittel sowie als Booster von kationisch modifizierten Guarderivaten.

### Stand der Technik

Kosmetische Zubereitungen für Haut- und Haar, wie Haarshampoos, Haarkonditionierer oder flüssiger Handseife, werden gerne auch in ihrem optischen Erscheinungsbild verschieden formuliert. Meist werden Produkte gewünscht, die einen Glimmer- oder Perlglanzeffekt aufweisen, oder Produkte, die eine Weißtrübung zeigen, aber nichtglänzend sind und meist als "milchig" bezeichnet werden. Zur Erzielung des Effektes der Weißtrübung werden sogenannte "Trübungsmittel" zugesetzt. Für ein gleichmäßiges Erscheinungsbild müssen Trübungsmittel unter anderem feinteilig sein. Des Weiteren müssen sie in den kosmetischen Zubereitungen homogen verteilbar sein und auch bei Lagerung nicht austrüben oder gar separieren.

Typische Trübungsmittel sind feinteilige Polymer- bzw. Feststoffdispersionen, die im Wesentlichen nur einen Wachskörper und einen geeigneten Emulgator neben Wasser und/oder einem Polyol wie Glycerin enthalten. Die aus dem Stand der Technik bekannten Trübungsmittel basieren oft auf Copolymerisaten von Acyl- bzw. Methacrylsäure und Styrol als Wachskörper und sind nicht biologisch abbaubar.

Aus dem Deutschen Patent DE 19511572 C sind Trübungsmittelkonzentrate auf Basis von Wachskörpern, Zuckertensiden und Partialglyceriden bekannt, die eine gute biologische Abbaubarkeiten aufweisen, jedoch hinsichtlich der Feinteiligkeit verbesserungswürdig sind.

Aus der Internationalen Patentanmeldung WO 03/033634 sind niedrigviskose Trübungsmittel bekannt, die Fettsäurepolyglykolester, amphotere Tenside und noch Fettsäurepartialglyceride sowie gegebenenfalls Polyole in bestimmten, begrenzten Gewichtsverhältnissen enthalten. Die vorteilhaften Eigenschaften wie Feinteiligkeit, gute Fließ- und Pumpeigenschaften, niedrige Viskosität, gute Lagerstabilität und schöne Weißtrübung können nach dieser Internationalen Anmeldung nur erhalten werden, wenn keine anionischen Tenside zugegen sind. Bei Anwesenheit von anionischen Tensiden werden keine Trübungsmittel, sondern Perlglanzmittel erhalten und ein unakzeptabler Viskositätsanstieg erzeugt.

Demzufolge hat die Aufgabe der vorliegenden Erfindung darin bestanden, Wachsdispersionen auf Basis von Wachskörpern zur Verfügung zu stellen, die auch bei Anwesenheit von anionischen Tensiden, insbesondere von Fettalkohol(ether)sulfaten, eine Weißtrübung und keinen Perlglanz zeigen.

Des Weiteren sollten die Wachsdispersionen zusätzlich eine konditionierende Wirkung wie eine verbesserte Nass- und Trockenkämmbarkeit in Haarshampoos bzw. ein angenehmes, weiches Körpergefühl für Hautpflegemittel aufweisen und dies auch in Anwesenheit der weitverbreiteten anionischen Tenside.

Zudem galt es lagerstabile Wachsdispersionen zur Verfügung zu stellen, die lange Zeit unverändert die Weißtrübung in gleichbleibender Qualität zeigen und im Vergleich zum Stand der Technik eine sehr gute Homogenität und eine deutlich verminderte durchschnittliche Teilchengrößen (Feinteiligkeit) aufweisen.

### Beschreibung der Erfindung

Gegenstand der Erfindung sind wässrige Wachsdispersionen geeignet als Trübungs- und/oder Konditioniermittel für kosmetische Zubereitungen enthaltend
(a) Ethylenglykolfettsäureester als Wachskörper und
(b) Fettsäurepartialglyceride und
(c) anionische Tenside, ausgewählt aus der von Fettalkohol(ether)sulfaten gebildeten Gruppe und
(d) amphotere Tenside ausgewählt aus der von Betainen gebildeten Gruppe,
wobei die Ethylenglykolfettsäureester (a) gebildet sind von Stearinsäure oder einer Mischung von Fettsäuren, die 85-100 Gew.-% - bezogen auf Fettsäure - Stearinsäure enthalten.

Im Sinne der vorliegenden Erfindung sind "Konditioniermittel" Mittel auf dem Gebiet der kosmetischen Zubereitungen, die bei der Anwendung auf dem Haar und/oder der Haut eine konditionierende Wirkung zeigen.

Im Sinne der vorliegenden Erfindung sind Mittel konditionierend auf der Haut, wenn sie nach direktem Kontakt auf der menschlichen Haut ein positives Hautgefühl verursachen, die von Probanden in der Praxis wie in Panel-Tests mit Sinneseindrücken in Bezug auf bestimmte Parameter, wie "Trockenheit der Haut", "Weichheit der Haut" positiv bewertet werden.

Im Sinne der vorliegenden Erfindung sind die Mittel konditionierend für Haare, wenn sie nach deren Behandlung verbesserte Kämmbarkeiten zeigen. Die bessere Kämmbarkeit kann im nassen und/oder trockenen Zustand sowohl in den Längen als auch in den Spitzen (sog. Entwirrbarkeit) des Haares auftreten. Konditionierend wirkende Mittel verbessern auch taktile Eigenschaften der Haare wie Glätte, Weichheit, Geschmeidigkeit, Haarglanz, weniger elektrostatische Aufladung und bessere Frisierbarkeit.

Trübungsmittel, die auch als Weißmacher bzw. Opacifier bezeichnet werden, sind im Sinne der vorliegenden Erfindung Mittel, die eine weiße Trübung ohne Perlglanz erzeugen.

Im Sinne der vorliegenden Erfindung wurde die mittlere Teilchengröße (d50) und die Partikelgrößenverteilung in µm mittels Laserbeugung mit dem Gerät Mastersizer^{®} 2000 und der entsprechenden Produktbeschreibung der Firma MALVERN INSTRUMENTS GmbH, Marie -Curie-Straße 4/1, 71083 Herrenberg, Germany, bestimmt.

Die erfindungsgemäßen Wachsdispersionen enthalten als Wachskörper a) spezielle Ethylenglykolfettsäureester. Ethylenglykolfettsäureester folgen der allgemeinen Formel

RCO-OCH₂CH₂O-OCR

und sind herstellbar durch Veresterung von Ethylenglykol mit Fettsäuren bzw. Fettsäuremischungen. Die unter der INCI Bezeichnung Ethylenglykoldistearat bekannten Ethylenglykolfettsäureester weisen technisch unterschiedliche Veresterungsgrade und Fettsäureschnitte auf. Gemäß der vorliegenden Erfindung enthält die Fettsäuremischung 85 bis 100 Gew.-% Stearinsäure und damit deutlich höhere Anteile an Stearinsäure als die etwa 50 Gew.-% in handelsüblich technischen Mischungen, die beispielsweise unter den Handelsnamen Cutina^{®} AGS, von der BASF Personal Care Nutrition GmbH, verkauft werden.

Vorzugsweise enthält die Fettsäuremischung im Sinne der Erfindung 85 bis 100 Gew.-% Stearinsäure und 0 bis 15 Gew.-% andere Fettsäuren mit 12 bis 22 Kohlenstoffatomen - bezogen auf die gesamte Menge an Fettsäure. Bevorzugt enthält die Fettsäuremischung 90 bis 98 Gew.-% Stearinsäure und 2 bis 10 Gew.-% andere gesättigte Fettsäuren mit 16 bis 22 Kohlenstoffatomen - bezogen auf die gesamte Menge an Fettsäure und insbesondere besteht die Fettsäuremischung aus 90 bis 96 Gew.-% Stearinsäure und 4 bis 10 Gew.-% andere gesättigte Fettsäuren mit 16 bis 22 Kohlenstoffatomen. Andere gesättigte Fettsäuren mit 16 bis 22 Kohlenstoffatomen sind Palmitinsäure, Arachinsäure und Behensäure

Im Sinne der Erfindung bevorzugte Ethylenglykolfettsäureester sind Ethylenglykoldifettsäureester, die technisch bedingt 90 bis 100% Ethylenglykoldisäureester und 0 bis 10 Gew.-% Ethylenglykolmonofettsäureester enthalten.

Derartige Ethylenglykolfettsäureester mit bevorzugten Ethylenglykoldifettsäuregehalt und Stearinsäuregehalt in der Fettsäuremischung sind im Markt unter dem Markennamen Cutina^{®} KE2747, BASF, erhältlich.

Innerhalb der Wachskörper a) sind solche Ethylenglykolfettsäureester ganz besonders geeignet, die einen Schmelzpunkt im Bereich von 72 bis 80 °C aufweisen.

Für die erfindungsgemäßen Wachsdispersionen empfiehlt es sich, dass die Wachskörper a) in Mengen von 20 bis 35, vorzugsweise 25 bis 30 Gew.-% - bezogen auf die Wachsdispersion - enthalten sind.

Vorzugsweise hat in den erfindungsgemäßen Wachsdispersionen a) einen mittleren Teilchendurchmesser im Bereich von 1 bis 4 µm. Bevorzugt ist die Verteilung der Teilchendurchmesser so, dass mehr als 50% unter 3 µm und 90% aller Teilchen unter 7 µm liegen.

Insbesondere sind Wachskörper a) geeignet, die eine überwiegend sphärische Teilchenform zeigen, wobei 70% aller Teilchen eine dreidimensionale Struktur aufweisen, die ganz besonders durch ein Verhältnis von Höhe: Breite: Länge von 1: 1: 1 gekennzeichnet sind. Derartige Teilchenformen und Teilchenmengen sind mittels Laserbeugung mit dem Gerät Mastersizer^{®} 2000 und der entsprechenden Produktbeschreibung der Firma MALVERN INSTRUMENTS GmbH, Marie-Curie-Straße 4/1, 71083 Herrenberg, Germany, bestimmbar.

Als weitere Komponente b) enthalten die Wachsdispersionen Fettsäurepartialglyceride. Fettsäurepartialglyceride sind im Sinne der Erfindung Monoglyceride, Diglyceride und deren technische Gemische, die herstellungsbedingt noch geringe Mengen an Glycerin und Triglyceride enthalten können. Bevorzugt sind als b) technische Mischungen von Fettsäurepartialglyceriden, die einen Monoglyceridanteil im Bereich von 50 bis 95 Gew.-%, vorzugsweise 60 bis 90 Gew.-%, aufweisen und im Folgenden auch als Glycerinmonofettsäureester bezeichnet werden.

Im Sinne der vorliegenden Erfindung werden Fettsäurepartialglyceride (b) bevorzugt, die ausgewählt sind aus der von der Glycerinmonofettsäureester gebildeten Gruppe, wobei als Fettsäuren Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen geeignet sind. Besonders bevorzugt sind Glycerinmonofettsäureester, wobei die Fettsäuren 12 bis 18 C-Atome aufweisen, vorzugsweise ist (b) ein Glycerinmonofettsäureester von einer Fettsäuremischung, die 90 bis 100 Gew.-% Ölsäure enthält - bezogen auf Fettsäuremischung.

Ein geeignetes technisches Produkt im Markt ist beispielsweise Monomuls^{®} 90-O 18, ein Handelsprodukt der BASF Personal Care & Nutrition GmbH.

Die Fettsäurepartialglyceride b) sind vorzugsweise in Mengen von 0,5 bis 3,0, bevorzugt 1,0 bis 2 Gew.-% - bezogen auf die wässrige Dispersion - enthalten.

Wässrige Wachsdispersionen mit dem besonders bevorzugten Glycerinmonooleat zeigen besonders niedrige Viskositäten, was für Verarbeitung, Pumpbarkeit vorteilhaft ist.

Hinsichtlich der Trübung und der Konditionierungseigenschaften ist es vorteilhaft, wenn die wässrigen Wachsdispersionen a) und b) in einem Gewichtsverhältnis von a): b) im Bereich von 15: 1 bis 20: 1, vorzugsweise 17:1 bis 18,5: 1 in der Dispersion enthalten.

Als Bestandteil c) sind in den Wachsdispersionen anionische Tenside enthalten, wobei diese ausgewählt aus der von Fettalkohol(ether)sulfaten gebildeten Gruppe sind.

Bevorzugt sind insbesondere Fettalkohol(ether)sulfate der allgemeinen Formel

R'O-(CH₂CH₂O)₀₋₁₀SO₃Y,

in der R' für einen linearen oder verzweigten Alkyl- und/oder Alkenylrest mit 6 bis 22 Kohlenstoffatomen und Y für ein Alkali- und/oder Erdalkalimetall, Ammonium, Alkylammonium, Alkanolammonium oder Glucammonium steht. Sofern die Zahl Null (=0) ist, handelt es sich um Fettalkoholsulfat. Bevorzugt sind Fettalkoholethersulfate (Zahl von 1 bis 10) enthalten. Typische Beispiele sind die Sulfate von Anlagerungsprodukten von durchschnittlich 1 bis 10 und insbesondere 2 bis 5 Mol Ethylenoxid an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen in Form ihrer Natrium- und/oder Magnesiumsalze. Die Ethersulfate können dabei sowohl eine konventionelle als auch eine eingeengte Homologenverteilung aufweisen.

Besonders bevorzugt sind im Sinne der Erfindung Wachsdispersionen, die als c) Laurylethersulfat mit einem durchschnittlichen Ethoxylierungsgrad von 1 oder 2 enthalten.

Vorzugsweise ist c) in den wässrigen Wachsdispersionen in einer Menge von 8 bis 15, vorzugsweise 9 bis 12 Gew.-% - bezogen auf wässrige Dispersion - enthalten.

Eine besonders gleichmäßige Teilchenverteilung wird in der Wachsdispersion erhalten, wenn a) und c) vorteilhafterweise in einem Gewichtsverhältnis im Bereich a): c) von 2: 1 bis 4: 1 und insbesondere im Bereich von 2,5: 1 bis 3,5: 1, enthalten sind.

Überraschenderweise wird trotz der Anwesenheit der anionischen Tenside kein Perlglanz und kein Anstieg der Viskosität der Wachsdispersionen beobachtet.

Als weiteren zwingenden Bestandteil enthalten die wässrigen Wachsdispersionen d) amphotere Tenside ausgewählt aus der von Betaine gebildeten Gruppe.

Geeignete Betaine sind beispielsweise Alkylbetaine, Alkylamidobetaine, Alkylglycinate, Imidazoliniumbetaine und Sulfobetaine. Betaine stellen bekannte Tenside dar, die überwiegend durch Carboxyalkylierung, vorzugsweise Carboxymethylierung von aminischen Verbindungen hergestellt werden. Vorzugsweise werden die Ausgangsstoffe mit Halogencarbonsäuren oder deren Salzen, insbesondere mit Natriumchloracetat kondensiert, wobei pro Mol Betain ein Mol Salz gebildet wird. Ferner ist auch die Anlagerung von ungesättigten Carbonsäuren, wie beispielsweise Acrylsäure möglich. Beispiele für geeignete Betaine stellen die Carboxyalkylierungsprodukte von sekundären und insbesondere tertiären Aminen dar, die der Formel (I) folgen, 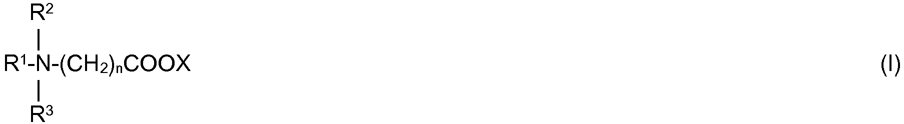 in der R¹ für Alkyl- und/oder Alkenylreste mit 6 bis 22 Kohlenstoffatomen, R² für Wasserstoff oder für Alkylreste mit 1 bis 4 Kohlenstoffatomen, R³ für Alkylreste mit 1 bis 4 Kohlenstoffatomen, n für Zahlen von 1 bis 6 und X für ein Alkali- und/oder Erdalkalimetall oder Ammonium steht.

Typische Beispiele sind die Carboxymethylierungsprodukte von Hexylmethylamin, Hexyldimethylamin, Octyldimethylamin, Decyldimethylamin, Dodecylmethylamin, Dodecyldimethylamin, Dodecylethylmethylamin, C_{12/14}-Kokosalkyldimethylamin, Myristyldimethylamin, Cetyldimethylamin, Stearyldimethylamin, Stearylethylmethylamin, Oleyldimethylamin, C_{16/18}-Talgalkyldimethyl-amin sowie deren technische Gemische.

Weiterhin kommen auch Carboxyalkylierungsprodukte von **Amidoaminen** in Betracht, die der Formel (II) folgen,

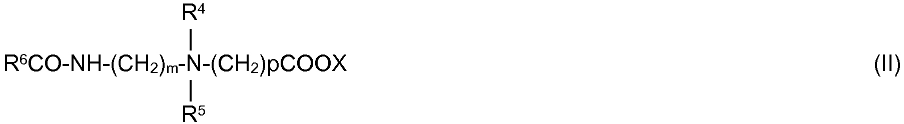

in der R⁶CO für einen aliphatischen Acylrest mit 6 bis 22 Kohlenstoffatomen und 0 oder 1 bis 3 Doppelbindungen, m für Zahlen von 1 bis 3 steht, R⁴ für Wasserstoff oder Alkylreste mit 1 bis 4 Kohlenstoffatomen, R⁵ für Alkylreste mit 1 bis 4 Kohlenstoffatomen, p für Zahlen von 1 bis 6 und X für ein Alkali- und/oder Erdalkalimetall oder Ammonium steht.

Typische Beispiele sind Umsetzungsprodukte von Fettsäuren R⁶COOH mit 6 bis 22 Kohlenstoffatomen, namentlich Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Gemische, mit N,N-Dimethylaminoethylamin, N,N-Dimethylaminopropylamin, N,N-Diethylaminoethylamin und N,N-Diethylaminopropylamin, die mit Natriumchloracetat kondensiert werden. Bevorzugt ist der Einsatz eines Kondensationsproduktes von C_{8/18}-Kokosfettsäure-N,N-dimethylaminopropylamid mit Natriumchloracetat.

Weiterhin kommen als geeignete Ausgangsstoffe für die im Sinne der Erfindung einzusetzenden Betaine auch Imidazoline in Betracht, die der Formel (III) folgen,

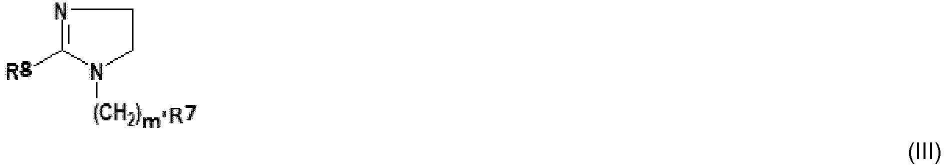

in der R⁷ für einen Alkylrest mit 5 bis 21 Kohlenstoffatomen, R⁸ für eine Hydroxylgruppe, einen O-COR⁹- oder NH-COR⁹-Rest, m' für 2 oder 3 und R⁹CO für einen aliphatischen Acylrest mit 6 bis 22 Kohlenstoffatomen und 0 oder 1 bis 3 Doppelbindungen steht. Auch bei diesen Substanzen handelt es sich um bekannte Stoffe, die beispielsweise durch cyclisierende Kondensation von 1 oder 2 Mol Fettsäure mit mehrwertigen Aminen, wie beispielsweise Aminoethylethanolamin (AEEA) oder Diethylentriamin erhalten werden können. Die entsprechenden Carboxyalkylierungsprodukte stellen Gemische unterschiedlicher offenkettiger Betaine dar. Typische Beispiele sind Kondensationsprodukte der Fettsäuren R⁹COOH, beispielsweise der oben genannten Fettsäuren R⁶COOH, mit AEEA, vorzugsweise Imidazoline auf Basis von Laurinsäure oder wiederum C_{12/14}-Kokosfett-säure, die anschließend mit Natriumchloracetat betainisiert werden.

Ein besonders geeignetes amphoteres Tensid d) ist Kokos(fettsäureamidopropyl)betain, welches unter der INCI-Bezeichnung *Cocamidopropyl Betaine* bekannt ist.

Die erfindungsgemäßen Wachsdispersionen enthalten d) in einer Menge von 0,5 bis 3,0 Gew.-%, vorzugsweise 1 bis 2,5 Gew.-% - bezogen auf wässrige Dispersion.

Besonders vorteilhaft sind Wachsdispersionen, die c) und d) in einem Gewichtsverhältnis von c): d) von 4: 1 bis 8: 1, vorzugsweise 5,5: 1 bis 7,5: 1 enthalten. So zeigen Wachsdispersionen mit einem derartigen Gewichtsverhältnis insbesondere eine hervorragende Lagerstabilität.

Eine bevorzugte wässrige Wachsdispersion enthält
a) 25 bis 30 Gew.-% Ethylenglykoldifettsäureester, wobei 85 bis 100 Gew.-% der Fettsäure Stearinsäure ist,
b) 1,0 bis 2,0 Gew.-% Glycerinmonofettsäureester, wobei 90 bis 100 Gew.-% der Fettsäure Ölsäure ist,
c) 9,0 bis 12 Gew.-% Laurylethersulfat mit einem durchschnittlichen Ethoxylierungsgrad von 1 oder 2
d) 1,0 bis 2,5 Gew.-% Kokosfettsäureamidopropylbetain.

Die Gewichtsangaben sind hier bezogen auf Aktivsubstanz.

Einer Ausführungsform der vorliegenden Erfindung entsprechend bestehen die erfindungsgemäßen wässrigen Wachsdisperionen aus den Bestandteilen a) bis d) und Wasser sowie pH-Regulatien und ggf. Konservierungsmittel.

Als Konservierungsmittel eignen sich beispielsweise Benzoate, Phenoxyethanol, Formaldehydlösung, Parabene, Pentandiol, Sorbinsäure, Lävulinsäure und Arachidonsäure sowie die unter der Bezeichnung Surfacine^{®} bekannten Silberkomplexe und die in Anlage 6, Teil A und B der Kosmetikverordnung aufgeführten weiteren Stoffklassen. Als pH-Wert-Regulatien eignen sich beispielsweise die in der kosmetischen Industrie bekannten, verträglichen Säuren bzw. Basen, die ebenso in der Kosmetikverordnung aufgeführt sind.

Insbesondere bevorzugt sind wässrige Wachsdispersion bestehend aus
a) 25 bis 30 Gew.-% Ethylenglykoldifettsäureester, wobei 85 bis 100 Gew.-% der Fettsäure Stearinsäure ist,
b) 1,0 bis 2,0 Gew.-% Glycerinmonofettsäureester, wobei 90 bis 100 Gew.-% der Fettsäure Ölsäure ist,
c) 9,0 bis 12 Gew.-% Laurylethersulfat mit einem durchschnittlichen Ethoxylierungsgrad von 1 oder 2
d) 1,0 bis 2,5 Gew.-% Kokosfettsäureamidopropylbetain
e) 0,1 bis 3,0 Gew.-% pH-Regulatien
f) 0 bis 1,5 Gew.-% Konservierungsmittel
g) ad 100 Gew.-% Wasser.

Die Wachsdispersionen zeigen niedrige Viskositäten, vorzugsweise unter 8000 und insbesondere 3000 bis 6000 mPas (nach Brookfield: 23 °C, Spindel 5, 10 Upm), gute Fließ- und Pumpeigenschaften sowie eine besondere Feinteiligkeit der Wachse in der Dispersion, beispielsweise erkennbar an dem durchschnittlichen Teilchendurchmesser und der Partikelgrößenverteilung.

Ohne an eine Theorie gebunden zu sein, scheint für das Trübungsverhalten und für die konditionierenden Effekte besonders die Auswahl der Wachskörper a) mit dem besonderen Fettsäureschnitt In Kombination mit dem Fettsäurepartialglycerid und deren ausgewähltem Gewichtsverhältnis von a): b) entscheidend zu sein. Eine besonders gleichmäßige Teilchenverteilung wird in der Wachsdispersion erhalten, wenn a) und c) vorteilhafterweise in einem Gewichtsverhältnis im Bereich a): c) von 2: 1 bis 4: 1 und insbesondere im Bereich von 2,5: 1 bis 3,5: 1, enthalten sind.

Ein weiterer Gegenstand der vorliegenden Erfindung betrifft daher die Verwendung der erfindungsgemäßen wässrigen Wachsdispersionen nach einem der Patentansprüche 1 bis 14 als Trübungsmittel in einer kosmetischen Zubereitung für die Haarbehandlung.

Ein weiterer Gegenstand der vorliegenden Erfindung betrifft die Verwendung der erfindungsgemäßen Wachsdispersionen nach einem der Patentansprüche 1 bis 14 als Konditionierungsmittel, insbesondere zur Verbesserung der Nass- und Trockenkämmbarkeit, in einer kosmetischen Zubereitung für die Haarbehandlung.

Als kosmetische Zubereitungen für die Haarbehandlung sind Haarshampoos, Haarlotionen, Haarkuren, Haarspülungen, Fönlotionen, Haarmilch oder auch Zubereitungen für die Pflege von Augenbrauen und Wimpern zu verstehen.

Die erfindungsgemäßen Wachsdispersionen werden vorzugsweise in Mengen von 1 bis 5 Gew.-% - bezogen auf kosmetische Zubereitung für die Haarbehandlung - eingesetzt.

Die erfindungsgemäßen Wachsdispersionen rufen eine dauerhafte, gleichmäßige und im Vergleich zum Stand der Technik eine besonders intensive Weißtrübung hervor, ohne dass dabei Perlglanz erzeugt wird und vor allem zeigen sie auch überragende Konditioniereffekte, so dass je nach Wunsch ganz oder teilweise auf weitere konditionierend wirkende Bestandteile in den kosmetischen Zubereitungen für die Haarpflege verzichtet werden kann.

Nach einer weiteren Ausführungsform der vorliegenden Erfindung ist es aber auch möglich, die erfindungsgemäßen Wachsdispersionen als Booster für kationisch modifizierten Polymere hinsichtlich der konditionierenden Wirkung einzusetzen.

Ein weiterer Gegenstand betrifft daher die Verwendung der wässrigen Wachsdispersionen nach einem der Ansprüche 1 bis 14 als Booster zur Verbesserung der konditionierenden Wirkung von kationisch modifizierten Polymeren in einer kosmetischen Zubereitung für die Haarbehandlung. Geeignete kationische Polymere sind beispielsweise die quaternierte Hydroxyethylcellulose, die auch unter der Bezeichnung UCAREPolymer^{®} JR 400 von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat^{®} (BASF), Kondensationsprodukte von Polyglycolen und Aminen, quaternierte Proteinhydrolysate, Polypeptide und Aminsäuren wie beispielsweise Lauryldimonium hydroxypropyl hydrolyzed collagen (Lamequat^{®}L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine^{®}/Sandoz), Copolymere der Acrylsäure mit Dimethyldiallylammoniumchlorid (Merquat^{®} 550/Chemviron), Polyaminopolyamide, wie z.B. beschrieben in der FR-A 2252840 sowie deren vernetzte wasserlöslichen Polymere, kationische Chitinderivate, wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar^{®} CBS, Jaguar^{®} C-17, Jaguar^{®} C-16 der Firma Celanese, quaternierte Ammoniumsalz-Polymere, wie z.B. Mirapol^{®} A-15, Mirapol^{®} AD-1, Mirapol^{®} AZ-1 der Firma Miranol. Insbesondere geeignete kationische Polymere sind Polyquaternium-68, erhältlich als Luviquat^{®} Supreme AT 1, oder Polyquaternium-11, erhältlich als Luviquat^{®} PQ 11 AT 1.

Die Wachsdispersionen boosten, d.h. verstärken die konditionierende Wirkung vorzugsweise von kationischen Polymeren ausgewählt aus der von kationisch modifiziertem Guar, PQ-7 und PQ-10 gebildeten Gruppe.

Kationisch modifizierte Guarderivate wie das quaternierte Guarderivat Guar Hydroxypropyltrimonium Chlorid sind beispielsweise im Handel als Dehyquart^{®} Guar N von der BASF Personal Care & Nutrition GmbH erhältlich. PQ-10 ist als kationisches Cellulosederivate unter der Bezeichnung UCARE^{®} Polymer JR 400 von Amerchol erhältlich und das als PQ-7 bekannte Copolymere von Diallylammoniumsalzen und Acrylamiden ist im Handel als Dehyquart^{®} CC7 der BASF Personal Care & Nutrition GmbH erhältlich.

Gemäß einer weiteren Ausführungsform der vorliegenden Erfindung werden kosmetische Mittel zur konditionierenden Haarbehandlung beansprucht, die die erfindungsgemäßen Wachsdispersionen, anionische Tenside und kationische Polymere sowie optional amphotere und/oder zwitterionische Tenside und Emulgatoren oder weitere übliche Inhaltsstoffe sowie zur Ergänzung auf 100 Gew.-% Wasser enthalten.

Vorzugsweise sind in den konditionierenden Haarbehandlungsmittel - bezogen auf Aktivsubstanzgehalt - enthalten
0,5 bis 5,0 Gew.-%, vorzugsweise 1,0 bis 3 Gew.-%, erfindungsgemäße Wachsdispersion
1,0 bis 15 Gew.-%, vorzugsweise 7,5 bis 12 Gew.-%, anionische Tenside
0,01 bis 1 Gew.-%, vorzugsweise 0,05 bis 0,4 Gew.-%, mindestens eines kationisch modifizierten Polymers und/oder
0,0 bis 15 Gew.-%, vorzugsweise 0,1 bis 5 Gew.-%, amphotere und/oder zwitterionische Tenside,
0,0 bis 10 Gew.-% Emulgatoren und optional weitere übliche Inhaltsstoffe enthalten sein können und zur Ergänzung auf 100 Gew.-% Wasser.

Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Alkylethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Alk(en)ylpolyglykolethercitraten bzw. deren Salzen, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Besonders bevorzugt werden als anionische Tenside die Fettalkohol(ethersulfate), die bereits im Zusammenahng mit den erfindungsgemäßen Wachsdispersionen beschrieben wurden und insbesondere Laurylethersulfat mit 1 oder 2 Mol Ethylenoxid.

Typische kationische Polymere sind bereits im Vorstehenden mit der Verwendung der Wachsdispersionen als Booster beschrieben worden und sind vorzugsweise ausgewählt aus der von kationisch modifiziertem Guar, PQ-7 und PQ-10 gebildeten Gruppe. Wobei auch Mischungen von modifiziertem Guar, PQ-7 und PQ-10 als kationisch modifizierte Polymere enthalten sein können, vorzugsweise Mischungen von kationisch modifiziertem Guar und PQ-10 und insbesondere im Gewichtsverhältnis 1:1.

Geeignete amphotere bzw. zwitterionische Tenside sind Alkylbetaine, Alkylamidobetaine, Aminopropionate, Aminoglycinate, Imidazoliniumbetaine und Sulfobetaine. Beispiele für geeignete Alkylbetaine stellen die Carboxyalkylierungsprodukte von sekundären und insbesondere tertiären Aminen dar. Typische Beispiele sind die Carboxymethylierungsprodukte von Hexylmethylamin, Hexyldimethylamin, Octyldimethylamin, Decyldimethylamin, Dodecylmethylamin, Dodecyldimethylamin, Dodecylethylmethylamin, C_{12/14}-Kokosalkyldimethylamin, Myristyldimethylamin, Cetyldimethylamin, Stearyldimethylamin, Stearylethylmethylamin, Oleyldimethylamin, C_{16/18}-Talgalkyldimethylamin sowie deren technische Gemische. Weiterhin kommen auch Carboxyalkylierungsprodukte von Amidoaminen in Betracht. Typische Beispiele sind Umsetzungsprodukte von Fettsäuren mit 6 bis 22 Kohlenstoffatomen, namentlich Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Gemische, mit N,N-Dimethylaminoethylamin, N,N-Dimethylaminopropylamin, N,N-Diethylaminoethylamin und N,N-Diethylaminopropylamin, die mit Natriumchloracetat kondensiert werden. Bevorzugt ist der Einsatz eines Kondensationsproduktes von C_{8/18}-Kokosfettsäure-N, N-dimethylaminopropylamid mit Natriumchloracetat.

Weiterhin kommen auch Imidazoliniumbetaine in Betracht. Auch bei diesen Substanzen handelt es sich um bekannte Stoffe, die beispielsweise durch cyclisierende Kondensation von 1 oder 2 Mol Fettsäure mit mehrwertigen Aminen wie beispielsweise Aminoethylethanolamin (AEEA) oder Diethylentriamin erhalten werden können. Die entsprechenden Carboxyalkylierungsprodukte stellen Gemische unterschiedlicher offenkettiger Betaine dar. Typische Beispiele sind Kondensationsprodukte der oben genannten Fettsäuren mit AEEA, vorzugsweise Imidazoline auf Basis von Laurinsäure oder wiederum C_{12/14}-Kokosfettsäure, die anschließend mit Natriumchloracetat betainisiert werden.

### Emulgatoren

Als Emulgatoren kommen beispielsweise nichtionogene Tenside aus mindestens einer der folgenden Gruppen in Frage:
- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Addukte mit 1 bis 30 Mol Ethylenoxid an Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen;
- Partialester von Polyglycerin (durchschnittlicher Eigenkondensationsgrad 2 bis 8), Polyethylenglycol (Molekulargewicht 400 bis 5000), Sorbitan, Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
- Mono-, Di- und Trialkylphosphate sowie Mono-, Di- und/oder Tri-PEG-alkylphosphate und deren Salze;
- Wollwachsalkohole;
- Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen-Typen (TR-1,TR-2) von Goodrich;
- Polyalkylenglycole sowie
- Glycerincarbonat.

Besonders bevorzugte Emulgatoren sind Anlagerungsprodukte von Ethylenoxid an C_{12/18}-Fettsäuremono- und -diester, Anlagerungsprodukte von 1 bis 30, vorzugsweise 5 bis 10 Mol Ethylenoxid an Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische. Ebenfalls geeignet sind Anlagerungsprodukte von 1 bis 30, vorzugsweise 5 bis 10 Mol Ethylenoxid an Sorbitanester. Als Sorbitanester kommen Sorbitanmonoisostearat, Sorbitansesquiisostearat, Sorbitan-diisostearat, Sorbitantriisostearat, Sorbitanmonooleat, Sorbitansesquioleat, Sorbitan-dioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesqui-tartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitan-dimaleat, Sorbitantrimaleat sowie deren technische Gemische in Frage. Typische Beispiele für geeignete Polyglycerinester sind Polyglyceryl-2 Dipolyhydroxystearate (Dehymuls^{®} PGPH), Polyglycerin-3-Diisostearate (Lameform^{®} TGI), Polyglyceryl-4 Isostearate (Isolan^{®} Gl 34), Polyglyceryl-3 Oleate, Diisostearoyl Polyglyceryl-3 Diisostearate (Isolan^{®} PDI), Polyglyceryl-3 Methylglucose Distearate (Tego Care^{®} 450), Polyglyceryl-3 Beeswax (Cera Beilina^{®}), Polyglyceryl-4 Caprate (Polyglycerol Caprate T2010/90), Polyglyceryl-3 Cetyl Ether (Chimexane^{®} NL), Polyglyceryl-3 Distearate (Cremophor^{®} GS 32) und Polyglyceryl Polyricinoleate (Admul^{®} WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen. Bevorzugt ist auch Trimethylpropan-EO/PO-Trioleat, ein Gemisch erhältlich durch die Umsetzung von Trimethylolpropantrioleat mit Ethylenoxid und Propylenoxid unter alkalischen Bedingungen. Dabei findet, zumindest zum Teil, eine Einlagerung der Ethylenoxideinheiten (EO) und der Propylenoxideinheiten (PO) in die Estergruppen des Trimethylolpropantrioleat statt. Das Trimethylpropan-EO/PO-Trioleat wird charakterisiert durch seinen Gehalt an EO- und PO-Einheiten pro Molekül im statistischen Mittel. In einer Ausführunsgform der vorliegenden Erfindung wird Trimethylpropan-EO/PO-Trioleat, mit 120 Ethylenoxideinheiten (EO) und 10 Propylenoxideinheiten (PO) eingesetzt.

Weitere übliche Inhaltsstoffe können sein Konsistenzgeber, Verdickungsmittel, Überfettungsmittel, Stabilisatoren, UV-Schutzmittel, biogene Wirkstoffe und Antioxidantien, Antischuppenwirkstoffe, Filmbildner, Proteinhydrolysate, Hydrotrope, Konservierungsmittel, Parfümöle, Aromen und Farbmittel.

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole^{®} und Pemulen-Typen von Goodrich; Synthalene^{®} von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone^{®} Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als Stabilisatoren können Metallsalze von Fettsäuren, wie z.B. Magnesium-, Aluminium- und/oder Zinkstearat bzw. -ricinoleat eingesetzt werden.

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methyl-benzyliden)campher beschrieben;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyl Triazon oder Dioctyl Butamido Triazone (Uvasorb^{®} HEB);
- Propan-1,3-dione, wie z.B. 1-(4-tert.Butylphenyl)-3-(4'methoxyphenyl)propan-1,3-dion;
- Ketotricyclo(5.2.1.0)decan-Derivate.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol^{®} 1789), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans, z.B. 4-tert.-Butyl-4'-methoxydibenzoylmethan (Parsol^{®} 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex^{®} T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet.

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt oder Vitaminkomplexe zu verstehen.

Antioxidantien unterbrechen die photochemische Reaktionskette, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival^{®} (Climbazole), Ketoconazol^{®}, (4-Acetyl-1-{-4-[2-(2.4-dichlorphenyl)r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon^{®} UD (Protein-Undecylensäurekondensat), Zinkpyrithion, Aluminiumpyrithion und Magnesiumpyrithion / Dipyrithion-Magnesiumsulfat in Frage.

Falls gewünscht können aus dem Stand der Technik bekannte Proteinhydrolysate eingesetzt werden, beispielsweise auf Basis von Keratin wie das im Handel erhältliche Nutrilan^{®} Keratin W PP oder auf Basis von Weizen wie Gluadin^{®} WLM Benz, Gluadin^{®} WK oder Gluadin^{®} WP. Es ist auch, möglich geringe Mengen an freien Aminosäuren wie Lysin oder Arginin zuzugeben. Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Als Konservierungsmittel eignen sich beispielsweise Benzoate, Phenoxyethanol, Formaldehydlösung, Parabene, Pentandiol, Sorbinsäure, Lävulinsäure und Arachidonsäure sowie die unter der Bezeichnung Surfacine^{®} bekannten Silberkomplexe und die in Anlage 6, Teil A und B der Kosmetikverordnung aufgeführten weiteren Stoffklassen.

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt. Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S.81-106 zusammengestellt sind. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

### Ausführungsbeispiele Wachsdispersionen:

Es wurden erfindungsgemäße Wachsdispersionen A1 bis A3 und eine Wachsdispersion zum Vergleich V1 hergestellt (Mengenangaben in Gew.-% Aktivsubstanz - bezogen auf Wachsdispersion). Hierzu wurden 2/3 des gesamten Wassers vorgelegt und auf 85 °C erhitzt. In die Heißphase wurden Ethylenglykoldistearat (erfindungsgemäß bzw. Vergleich), Texapon^{®} N701(= Natriumlaurylethersulfat + 1EO), Dehyton^{®} PK45 (= Kokos(fettsäureamidopropyl)betain) und das Monomuls^{®} (Fettsäurepartialglycerid) zugegeben und gerührt. Bei 64 °C wurde das letzte 1/3 des gesamten Wassers zugegeben und gerührt. Nach Abkühlen auf ca. 40 °C wurde Na-Benzoat, Na-Sulfat sowie Zitronensäure (50 gew.-%ige Lösung) zugegeben und nachgerührt.

Die erfindungsgemäßen Wachsdispersionen enthalten Cutina^{®} KE 2747 als Ethylenglykoldifettsäureester die Wachsdispersion zum Vergleich V1 enthält Cutina^{®} AGS mit folgender Spezifikation:
Zusammensetzung Ethylenglykoldistearat (Diester, Monoester und Fettsäure mit entsprechender C-Kettenverteilung zwischen C₁₆ und C₁₈ und C₁₈:

| | C-Kettenverteilung C₁₆ und C₁₈ | | | GC wt% | |
|---|---|---|---|---|---|
| | % C16+C20+C22 | % C18 | | Monoester | Diester |
| Erfindung Cutina^{®} KE 2747 | 2-10 | 90-98 | | 6 | 94 |
| Vergleich Cutina^{®} AGS | 48-52 | 48-52 | | 8 | 92 |

Die Zusammensetzung und die Eigenschaften der Wachsdispersionen sind in Tabelle 1 zu finden.

Die Wachsdispersion V2 enthält im Gegensatz zu den erfindungsgemäßen Wachsdispersionen kein Betain, sondern das nichtionische Tensid Laurylglucosid gemäß der DE 1951572C.

**Tabelle 1: Wachsdispersionen (Mengenangaben in Gew.-% Aktivsubstanz - bezogen auf die Endzusammensetzung -**

| **Zusammensetzung (INCI)** | **A1** | **A2** | **A3** | **V1** | **V2** |
|---|---|---|---|---|---|
| **Wachskörper (a)** | 27 | 27 | 27 | - | 27 |
| *Cutina^{®} KE 2747* | | | | | |
| Ethylenglycol Distearate | | | | | |
| **Wachskörper (a)** | - | - | - | 27 | |
| *Cutina^{®} AGS* | | | | | |
| Ethylenglycol Distearate | | | | | |
| **Monomuls^{®} 90-018 (b)** | 1,55 | - | - | 1,55 | |
| Glyceryl Oleate | | | | | |
| **Monomuls^{®} 90-L12 (b)** | - | - | 1,55 | - | 1,55 |
| Glyceryl Laurate | | | | | |
| **Monomuls^{®} 60-35C (b)** | - | 1,55 | - | - | |
| Glyceryl Stearate | | | | | |
| Natriumlaurylethersulfat | 10,15 | 10,15 | 10,15 | 10,15 | 10,15 |
| 1 EO **(c)** | | | | | |
| Plantacare^{®} 1200UP | - | - | - | - | 15,5 |
| Lauryl Glucoside | | | | | |
| **Cocoamidopropyl Betaine (d)** | 1,73 | 1,73 | 1,73 | 1,73 | - |
| Na-Benzoat | 0,5 | 0,5 | 0,5 | 0,5 | 0,5 |
| Zitronensäure 50%ig | 0,6 | 0,6 | 0,6 | 0,6 | |
| Wasser | Ad 100 | Ad100 | Ad100 | Ad100 | Ad 100 |
| ***pH*** | 4,7 | 4,7 | 4,7 | 4,7 | 4,7 |
| ***Gewichtsverhältnis a:b*** | 17,4:1 | 17,4:1 | 17,4:1 | 17,4:1 | 17,4:1 |
| ***Gewichtsverhältnis c:d*** | 6,5:1 | 6,5:1 | 6,5:1 | 6,5:1 | - |
| ***Gewichtsverhältnis a:c*** | 2,7:1 | 2,7:1 | 2,7:1 | 2,7:1 | 2,7:1 |
| ***Viskosität in mPas*** | 5500 | 8000 | 7200 | 15.500 | 35.000 |
| ***Partikelgröße d50 in µm*** | 1 | 2 | 1,7 | 5 | 11 |
| ***Partikelgröße d90 in µm*** | 3,5 | 6 | 5 | 12 | 19 |
| ***Mittelwert Teilchendurchmesser*** | 1,3 | 2,4 | 2 | 8,5 | 12 |
| ***Aussehen*** | trüb | trüb | trüb | Perlglanz | Perlglanz |

Die Feinteiligkeit wurde über Bestimmung der Partikelgrößenverteilung in µm und dem durchschnittlichen Teilchendurchmesser in µm mittels Laserbeugung (Mastersizer 2000) bestimmt (siehe Produktbeschreibung Firma MALVERN INSTRUMENTS GmbH, Marie-Curie-Straße 4/1, 71083 Herrenberg, Germany). Die Viskosität wurde nach der Brookfieldmethode (23 °C, Spindel 5, 10 Upm, mPas) ermittelt.

Aus Tabelle 1 wird ersichtlich, dass Wachsdispersionen mit Ethylenglykoldifettsäureester mit einem C18-Anteil von mindestens 90% (A1 bis A3) ein Trübungsmittel sind. Wachsdispersion A1 ist besonders niedrigviskos und hat eine kleine Teilchengröße. Wachsdispersionen mit einem Ethylenglykoldifettsäureester mit einem niederen C18-Anteil zeigen Perlglanzeffekt, höhere Viskosität und größeren Teilchendurchmesser (siehe V1). Wachsdispersionen mit Laurylglucosid anstelle von Betain als Tensid gemäß der DE 19511572C weisen auch einen Perlglanzeffekt, höhere Viskosität und größere Teilchengrößen auf (siehe V2).

### B) Verwendung der Wachsdispersionen in Haarshampoo-Formulierungen:

Durch Vermischen der folgenden Bestandteile bei 25 °C wurden wässrige, weiße Haarshampoo-Formulierungen mit folgenden Konditionier-Leistungen hergestellt:

**Tabelle 2: Menge (Gew.-%) as is**

| | Formulierungen | | | | | |
|---|---|---|---|---|---|---|
| | B1 | B2 | B3 | B4 (=Vergleich) | B5 (=Vergleich) | B6 (=Vergleich) |
| | Menge (Gew.-%) | | | | | |
| Wachsdispersion nach A1) | 2,20 | 2,20 | 2,20 | - | - | - |
| Dehyton PK^{®} 45 (INCI Cocoamidopropylbetain; 45%ig) | 5,40 | 5,40 | 5,40 | 5,40 | 5,40 | 5,40 |
| Texapon^{®} N70 (INCl: Sodium Laureth Sulfate+ 2EO 70%ig) | 14,30 | 14,30 | 14,30 | 14,30 | 14,30 | 14,30 |
| Dehyquart^{®}Guar N (INCI:Guar Hydroxypropyltrimonium Chloride; | 0,20 | 0,10 | | | | |
| UCARE Polymer ^{®}JR 400 (=INCI:Polyquaternium-10; | | 0,10 | | | | 0,20 |
| Euperlan^{®} PCO (=INCI: Styrene/ Acrylates Copolymer (and) Coco-Glucoside; 40 %ig) | | | | | 1,00 | |
| Glycerin (99,5 %) | 1,50 | 1,50 | 1,50 | 1,00 | 1,00 | 1,00 |
| Perfume "Cotton Touch" | 0,50 | 0,50 | 0,50 | 0,50 | 0,50 | 0,50 |
| Sodium Benzoat | 0,50 | 0,50 | 0,50 | 0,50 | 0,50 | 0,50 |
| Zitronensäure (50 %ig) | 0,65 | 0,65 | 0,61 | 0,73 | 0,62 | 0,66 |
| Natriumchlorid | 1,15 | 1,23 | 1,39 | 1,07 | 1,29 | 1,41 |
| Wasser | 73,60 | 73,52 | 73,60 | 76,30 | 75,19 | 76,03 |
| Nasskämmbarkeit | 56 (+-8) | 50 (+-6) | 56 (+-7) | 83 (+-8) | 93 (+-11) | 80 (+-11) |
| Trockenkämmbarkeit | 30 (+-3) | 27 (+-2) | 44 (+-10) | 68 (+-11) | 180 (+-43) | 124 (+-4) |
| Weißgrad (Schulnoten 1-6) | 1+ | 1 | 1 | transparent | 1 | transparent |

UCARE Polymer^{®} JR 400 ist ein sogenanntes Polyquaternium-10 (= PQ-10); Dehyquart^{®} Guar N ist ein kationisch modifierztes Guar Hydroxypropyltrimonium Chloride; beides sind kationische Polymere.

Für die Beurteilung des Weißgrades wurden Schulnoten vergeben im Vergleich zu B5, welches als Standard gesetzt wurde.

Die Nassrestkämmbarkeit/Trockenrestkämmbarkeit wurde wie folgt bestimmt:
- Vorbehandlung der Haarsträhnen:
   Nassrestkämmbarkeit: Die Haarsträhnen (Caucasian hair, 12 cm/1 g, International Hair Importers & Products, USA) wurden mit 6 % Natriumlaurylethersulfat-Lösung (Aktivsubstanzgehalt), pH 6.5, gereinigt und mit einer Lösung von 5 % Wasserstoffperoxid bei pH 9.4 für 20 Minuten gebleicht, gefolgt von einem intensiven Spülen der Haare.

Trockenrestkämmbarkeit: Die Haarsträhnen (Caucasian hair, 15 cm/2 g, International Hair Importers & Products, USA) wurden mit 6 % Natriumlaurylethersulfat-Lösung (Aktivsubstanzgehalt), pH 6.5, gereinigt und mit einer Lösung von 8,5 % Wasserstoffperoxid, pH 9.4 gebleicht für 20 Minuten, gefolgt von zweimaligem Spülen der Haare. Anschließend erfolgte 30 min Trocknung in einem Luftstrom bei max. 55 °C.
- Behandlung der Haarsträhnen mit den Formulierungen B1) bis B6)

In einem automatisierten Verfahren erfolgte zunächst eine Nullmessung und dann eine Messung nach der Behandlung der Haare mit den erfindungsgemäßen Formulierungen B1) bis B3) bzw. zum Vergleich mit B4) bis B6). Die Haare wurden dazu mit 0,25 g/g Haar für 5 Minuten behandelt. In dem automatisierten System der Nassauskämmapparatur wurde die behandelte Haarsträhne unter Standardbedingungen (38°C, 1 l/Minute) für 1 Minute ausgespült und gemessen.

Für die Bestimmung der Trockenkämmbarkeit wurden die behandelten Haarsträhnen für 16 Stunden bei 30°C und 40% relativer Luftfeuchtigkeit gelagert.

Für jede Bestimmung wurden je 10 Haarsträhnen getestet und die Kämmkräfte während 23 Kämmungen (3. bis 23.) gemessen.

Die %-Restkämmbarkeit wurde wie folgt kalkuliert: Restkämmarbeit (%) = (Kämmarbeit nach Produktbehandlung: Kämmarbeit vor Produktbehandlung) x100.

Aus der Tabelle zu B) wird deutlich, dass die Haarshampoo-Formulierungen B1+B2+B3 mit den Wachsdispersionen nach A1) eine bessere Nass-und Trockenrestkämmbarkeit zeigen als Formulierungen ohne Wachsdispersionen. Dies gilt auch, wenn in den Haarshampoo-Formulierungen kationische Polymere enthalten sind (siehe B1 ist besser als Vergleich B4). Hinsichtlich Nass-und Trockenrestkämmbarkeit sind die Haarshampoo-Formulierungen B1) und B2), die neben der Wachsdispersion A1 zusätzlich kationisch modifizierte Polymere enthalten, besonders gut. Demnach verhält sich die Wachsdispersion selber als Konditionierungsmittel (siehe B3) und ist auch ein Booster für kationisch modifizierte Polymere.

## Patentansprüche

1. Wässrige Wachsdispersionen geeignet als Trübungs- und/oder Konditioniermittel für kosmetische Zubereitungen enthaltend
a) Ethylenglykolfettsäureester als Wachskörper und
b) Fettsäurepartialglyceride und
c) anionische Tenside, ausgewählt aus der von Fettalkohol(ether)sulfaten gebildeten Gruppe und
d) amphotere Tenside ausgewählt aus der von Betainen gebildeten Gruppe,
wobei die Ethylenglykolfettsäureester (a) gebildet sind von Stearinsäure oder einer Mischung von Fettsäuren, die 85-100 Gew.-% - bezogen auf Fettsäure - Stearinsäure enthalten.

2. Wässrige Wachsdispersionen nach Anspruch 1, **dadurch gekennzeichnet, dass** als a) Ethylenglykolfettsäureester enthalten sind, gebildet aus 90 bis 98 Gew.-% Stearinsäure und 2 bis 10 Gew.-% andere gesättigte Fettsäuren mit 16 bis 22 Kohlenstoffatomen - bezogen auf die gesamte Menge an Fettsäure.

3. Wässrige Wachsdispersionen nach Anspruch 1 oder 2, **dadurch gekennzeichnet, dass** der mittlere Teilchendurchmesser von (a) im Bereich von 1 bis 4 µm liegt.

4. Wässrige Wachsdispersionen nach einem der Ansprüche 1 bis 3, **dadurch gekennzeichnet, dass** der Wachskörper a) in der Dispersion in einer Menge von 20 bis 35, vorzugsweise 25 bis 30 Gew.-% - bezogen auf wässrige Dispersion - enthalten ist.

5. Wässrige Wachsdispersionen nach einem der Ansprüche 1 bis 4, **dadurch gekennzeichnet, dass** die Fettsäurepartialglyceride (b) ausgewählt sind aus der von der Glycerinmonofettsäureester gebildeten Gruppe, wobei die Fettsäuren 12 bis 18 C-Atome aufweisen, vorzugsweise ist das Fettsäurepartialglycerid (b) ein Glycerinmonofettsäureester von einer Fettsäuremischung, die 90 bis 100 Gew.-% Ölsäure enthält - bezogen auf Fettsäuremischung.

6. Wässrige Wachsdispersionen nach einem der Ansprüche 1 bis 5, **dadurch gekennzeichnet, dass** die Fettsäurepartialglyceride (b) in einer Menge von 0,5 bis 3,0 vorzugsweise 1 bis 2 Gew.-% - bezogen auf wässrige Dispersion - enthalten sind.

7. Wässrige Wachsdispersionen nach einem der Ansprüche 1 bis 6, **dadurch gekennzeichnet, dass** a) und b) im Gewichtsverhältnis a): b) = 15: 1 bis 20: 1, vorzugsweise 17:1 bis 18,5: 1 in der Dispersion enthalten sind.

8. Wässrige Wachsdispersionen nach einem der Ansprüche 1 bis 7, **dadurch gekennzeichnet, dass** c) ausgewählt ist aus der von Laurylethersulfat mit einem durchschnittlichen Ethoxylierungsgrad von 1 bis 2 gebildeten Gruppe.

9. Wässrige Wachsdispersionen nach einem der Ansprüche 1 bis 8, **dadurch gekennzeichnet, dass** c) in einer Menge von 8 bis 15, vorzugsweise 9 bis 12 Gew.-% - bezogen auf wässrige Dispersion - enthalten ist.

10. Wässrige Wachsdispersionen nach einem der Ansprüche 1 bis 9, **dadurch gekennzeichnet, dass** a) und c) im Gewichtsverhältnis a): c) = 2: 1 bis 4: 1, vorzugsweise 2,5: 1 bis 3,5: 1 in der Dispersion enthalten sind.

11. Wässrige Wachsdispersionen nach einem der Ansprüche 1 bis 10, **dadurch gekennzeichnet, dass** d) ausgewählt ist aus der von Kokos(fettsäureamidopropyl)betain gebildeten Gruppe.

12. Wässrige Wachsdispersionen nach einem der Ansprüche 1 bis 11, **dadurch gekennzeichnet, dass** d) in einer Menge von 0,5 bis 3,0, vorzugsweise 1 bis 2,5 Gew.-% - bezogen auf wässrige Dispersion - enthalten ist.

13. Wässrige Wachsdispersionen nach einem der Ansprüche 1 bis 12, **dadurch gekennzeichnet, dass** c) und d) im Gewichtsverhältnis c): d) = 4: 1 bis 8: 1, vorzugsweise 5,5: 1 bis 7,5: 1 in der Dispersion enthalten sind.

14. Wässrige Wachsdispersionen nach einem der Ansprüche 1 bis 13, **dadurch gekennzeichnet, dass** diese bestehen aus a) bis d) und Wasser sowie pH-Regulatien und ggf. Konservierungsmittel.

15. Verwendung der wässrigen Wachsdispersionen nach einem der Ansprüche 1 bis 14 als Trübungsmittel in einer kosmetischen Zubereitung für die Haarbehandlung.

16. Verwendung der wässrigen Wachsdispersionen nach einem der Ansprüche 1 bis 14 als Konditionierungsmittel, insbesondere zur Verbesserung der Nass- und/oder Trockenkämmbarkeit, in einer kosmetischen Zubereitung für die Haarbehandlung.

17. Verwendung der wässrigen Wachsdispersionen nach einem der Ansprüche 1 bis 14 als Booster zur Verbesserung der konditionierenden Wirkung von kationisch modifizierten Polymeren in einer kosmetischen Zubereitung für die Haarbehandlung.

18. Verwendung der wässrigen Wachsdispersionen nach einem der Ansprüche 15 bis 17 in Mengen von 1 bis 5 Gew.-% - bezogen auf Zubereitung für die Haarbehandlung.

19. Verwendung der wässrigen Wachsdispersionen nach einem der Ansprüche 17 oder 18 **dadurch gekennzeichnet, dass** die Wachsdispersionen als Booster von kationisch modifizierten Polymeren ausgewählt aus der von kationisch modifiziertem Guar, PQ-7 und PQ-10 gebildeten Gruppe verwendet werden.

20. Kosmetische Mittel zur konditionierenden Haarbehandlungen enthaltend die wässrigen Wachsdispersionen nach einem der Ansprüche 1 bis 14 und anionische Tenside und kationische Polymere sowie optional amphotere/zwitterionische Tenside, Emulgatoren oder weitere übliche Inhaltsstoffe sowie zur Ergänzung auf 100 Gew.-% Wasser.

## Claims

1. An aqueous wax dispersion suitable as opacifier and/or conditioner for cosmetic formulations, comprising
a) ethylene glycol fatty acid esters as wax body and
b) fatty acid partial glycerides and
c) anionic surfactants selected from the group formed by fatty alcohol (ether) sulfates and
d) amphoteric surfactants selected from the group formed by betaines,
wherein the ethylene glycol fatty acid esters (a) are formed by stearic acid or a mixture of fatty acids comprising 85-100% by weight - based on fatty acid - of stearic acid.

2. The aqueous wax dispersion according to claim 1, wherein ethylene glycol fatty acid esters present as a) are formed from 90% to 98% by weight of stearic acid and 2% to 10% by weight of other saturated fatty acids having 16 to 22 carbon atoms - based on the total amount of fatty acid.

3. The aqueous wax dispersion according to claim 1 or 2, wherein the average particle diameter of (a) is in the range from 1 to 4 µm.

4. The aqueous wax dispersion according to any of claims 1 to 3, wherein the wax body a) is present in the dispersion in an amount of 20% to 35%, preferably 25% to 30%, by weight - based on aqueous dispersion.

5. The aqueous wax dispersion according to any of claims 1 to 4, wherein the fatty acid partial glycerides (b) are selected from the group formed by glycerol fatty acid monoesters, wherein the fatty acids have 12 to 18 carbon atoms, and the fatty acid partial glyceride (b) is preferably a glycerol fatty acid monoester of a fatty acid mixture comprising 90% to 100% by weight of oleic acid - based on fatty acid mixture.

6. The aqueous wax dispersion according to any of claims 1 to 5, wherein the fatty acid partial glycerides (b) are present in an amount of 0.5% to 3.0%, preferably 1% to 20, by weight - based on aqueous dispersion.

7. The aqueous wax dispersion according to any of claims 1 to 6, wherein a) and b) are present in the dispersion in a weight ratio of a) :b) = 15:1 to 20:1, preferably 17:1 to 18.5:1.

8. The aqueous wax dispersion according to any of claims 1 to 7, wherein c) is selected from the group formed by lauryl ether sulfate having an average ethoxylation level of 1 to 2.

9. The aqueous wax dispersion according to any of claims 1 to 8, wherein c) is present in an amount of 8% to 15%, preferably 9% to 12%, by weight - based on aqueous dispersion.

10. The aqueous wax dispersion according to any of claims 1 to 9, wherein a) and c) are present in the dispersion in a weight ratio of a):c) = 2:1 to 4:1, preferably 2.5:1 to 3.5:1.

11. The aqueous wax dispersion according to any of claims 1 to 10, wherein d) is selected from the group formed by coco(fatty acid amidopropyl) betaine.

12. The aqueous wax dispersion according to any of claims 1 to 11, wherein d) is present in an amount of 0.5% to 3.0%, preferably 1% to 2.5%, by weight - based on aqueous dispersion.

13. The aqueous wax dispersion according to any of claims 1 to 12, wherein c) and d) are present in the dispersion in a weight ratio of c):d) = 4:1 to 8:1, preferably 5.5:1 to 7.5:1.

14. The aqueous wax dispersion according to any of claims 1 to 13, which consists of a) to d) and water, and also pH regulators and optionally preservatives.

15. The use of the aqueous wax dispersion according to any of claims 1 to 14 as opacifier in a cosmetic formulation for hair treatment.

16. The use of the aqueous wax dispersion according to any of claims 1 to 14 as conditioner, especially for improvement of dry and/or wet combability, in a cosmetic formulation for hair treatment.

17. The use of the aqueous wax dispersion according to any of claims 1 to 14 as a booster for improving the conditioning action of cationically modified polymers in a cosmetic formulation for hair treatment.

18. The use of the aqueous wax dispersion according to any of claims 15 to 17 in amounts of 1% to 5% by weight - based on the formulation for hair treatment.

19. The use of the aqueous wax dispersion according to either of claims 17 and 18, wherein the wax dispersion is used as a booster for cationically modified polymers selected from the group formed by cationically modified guar, PQ-7 and PQ-10.

20. A cosmetic composition for conditioning hair treatments comprising the aqueous wax dispersion according to any of claims 1 to 14 and anionic surfactants and cationic polymers, and optionally amphoteric/zwitterionic surfactants, emulsifiers or further customary ingredients, and water for supplementation to 100% by weight.

## Revendications

1. Dispersions aqueuses de cire appropriées en tant qu'agent opacifiant et/ou de conditionnement pour des préparations cosmétiques, contenant
a) des esters d'acides gras d'éthylèneglycol en tant que corps cireux et
b) des glycérides partiels d'acides gras et
c) des tensioactifs anioniques, choisis dans le groupe formé par les (éther)sulfates d'alcools gras et
d) des tensioactifs amphotères choisis dans le groupe formés par les bétaïnes,
les esters d'acides gras d'éthylèneglycol (a) étant formés d'acide stéarique ou d'un mélange d'acides gras qui contiennent 85-100% en poids - par rapport aux acides gras - d'acide stéarique.

2. Dispersions aqueuses de cire selon la revendication 1, **caractérisées en ce qu'**elles contiennent, comme a), des esters d'acides gras d'éthylèneglycol, formés par 90 à 98% en poids d'acide stéarique et 2 à 10% en poids d'autres acides gras saturés comprenant 16 à 22 atomes de carbone - par rapport à la quantité totale d'acides gras.

3. Dispersions aqueuses de cire selon la revendication 1 ou 2, **caractérisées en ce que** le diamètre moyen de particule de (a) se situe dans la plage de 1 à 4 µm.

4. Dispersions aqueuses de cire selon l'une des revendications 1 à 3, **caractérisées en ce que** le corps cireux a) est contenu dans la dispersion en une quantité de 20 à 35, de préférence de 25 à 30% en poids - par rapport à la dispersion aqueuse.

5. Dispersions aqueuses de cire selon l'une des revendications 1 à 4, **caractérisées en ce que** les glycérides partiels d'acides gras (b) sont choisis dans le groupe formé par les esters de monoacides gras de glycérol, les acides gras présentant 12 à 18 atomes de carbone, de préférence, le glycéride partiel d'acides gras (b) est un ester de monoacides de glycérol d'un mélange d'acides gras, qui contient 90 à 100% en poids d'acide oléique - par rapport au mélange d'acides gras.

6. Dispersions aqueuses de cire selon l'une des revendications 1 à 5, **caractérisées en ce que** les glycérides partiels d'acides gras (b) sont contenus en une quantité de 0,5 à 3,0, de préférence de 1 à 2% en poids - par rapport à la dispersion aqueuse.

7. Dispersions aqueuses de cire selon l'une des revendications 1 à 6, **caractérisées en ce que** a) et b) sont contenus dans la dispersion dans le rapport pondéral a):b) = 15:1 à 20:1, de préférence 17:1 à 18,5:1.

8. Dispersions aqueuses de cire selon l'une des revendications 1 à 7, **caractérisées en ce que** c) est choisi dans le groupe formé par l'éthersulfate de lauryle présentant un degré d'éthoxylation de 1 à 2.

9. Dispersions aqueuses de cire selon l'une des revendications 1 à 8, **caractérisées en ce que** c) est contenu en une quantité de 8 à 15, de préférence de 9 à 12% en poids - par rapport à la dispersion aqueuse.

10. Dispersions aqueuses de cire selon l'une des revendications 1 à 9, **caractérisées en ce que** a) et c) sont contenus dans la dispersion dans un rapport pondéral a):c) = 2:1 à 4:1, de préférence 2,5:1 à 3,5:1.

11. Dispersions aqueuses de cire selon l'une des revendications 1 à 10, **caractérisées en ce que** d) est choisi dans le groupe formé par la ((acide gras)amidopropyl)bétaïne de coco.

12. Dispersions aqueuses de cire selon l'une des revendications 1 à 11, **caractérisées en ce que** d) est contenu en une quantité de 0,5 à 3,0, de préférence de 1 à 2,5% en poids - par rapport à la dispersion aqueuse.

13. Dispersions aqueuses de cire selon l'une des revendications 1 à 12, **caractérisées en ce que** c) et d) sont contenus dans la dispersion dans un rapport pondéral c):d) = 4:1 à 8:1, de préférence 5,5:1 à 7,5:1.

14. Dispersions aqueuses de cire selon l'une des revendications 1 à 13, **caractérisées en ce qu'**elles sont constituées de a) à d) et d'eau ainsi que d'agents de régulation du pH et le cas échéant de conservateurs.

15. Utilisation des dispersions aqueuses de cire selon l'une des revendications 1 à 14 en tant qu'opacifiant dans une préparation cosmétique pour le traitement des cheveux.

16. Utilisation des dispersions aqueuses de cire selon l'une des revendications 1 à 14 en tant qu'agent de conditionnement, en particulier pour l'amélioration de l'aptitude du peignage humide et/ou à sec dans une préparation cosmétique pour le traitement des cheveux.

17. Utilisation des dispersions aqueuses de cire selon l'une des revendications 1 à 14 en tant qu'amplificateur pour l'amélioration de l'effet de conditionnement de polymères modifiés cationiquement dans une préparation cosmétique pour le traitement des cheveux.

18. Utilisation des dispersions aqueuses de cire selon l'une des revendications 15 à 17 en des quantités de 1 à 5% en poids - par rapport à la préparation pour le traitement des cheveux.

19. Utilisation des dispersions aqueuses de cire selon l'une des revendications 17 ou 18, **caractérisée en ce que** les dispersions de cire sont utilisées en tant qu'amplificateur de polymères modifiés cationiquement choisis dans le groupe formé par le guar, le PQ-7 et le PQ-10 modifiés cationiquement.

20. Agent cosmétique destiné à des traitements de conditionnement des cheveux, contenant les dispersions aqueuses de cire selon l'une des revendications 1 à 14 et des tensioactifs anioniques et des polymères cationiques ainsi qu'éventuellement des tensioactifs amphotères/zwittérioniques, des émulsifiants ou d'autres constituants usuels ainsi que de l'eau pour compléter à 100% en poids.
